# EUROPÄISCHE PATENTSCHRIFT

(11) **EP 1 954 351 B1**
(45) Veröffentlichungstag und Bekanntmachung des Hinweises auf die Patenterteilung: **13.04.2011**
(21) Anmeldenummer: 06806861.8
(22) Anmeldetag: 28.09.2006
(51) Int. Cl.: A61N 5/10

(54) **EINRICHTUNG FÜR DIE RÖNTGEN-BRACHYTHERAPIE**
DEVICE FOR X-RAY BRACHYTHERAPY
DISPOSITIF DE BRACHYTHERAPIE AUX RAYONS X

(30) Priorität: 24.11.2005 DE 102005056066
(43) Veröffentlichungstag der Anmeldung: 13.08.2008
(73) Patentinhaber: SIEMENS AKTIENGESELLSCHAFT, 80333 München (DE)
(72) Erfinder: FEHRE, Jens, 91353 Hausen (DE); GRANZ, Bernd, 90522 Oberasbach (DE); LANSKI, Markus, 73433 Wasseralfingen (DE); NANKE, Ralf, 91077 Neunkirchen (DE)
(86) Internationale Anmeldenummer: PCT/EP2006/066834
(87) Internationale Veröffentlichungsnummer: WO 2007/060049

(56) Entgegenhaltungen:
- DE-B3-102004 008 373
- DE-C1- 19 731 307
- US-A1- 2004 126 142
- US-B1- 6 540 655

## Beschreibung

Einrichtung für die Röntgen-Brachytherapie mit einer in das Innere eines Körpers zur Röntgen-Brachytherapie einführbaren Sonde

Die Erfindung bezieht sich auf eine Einrichtung für die Röntgen-Brachytherapie mit einer in das Innere eines Körpers zur Röntgen-Brachytherapie einführbaren Sonde.

Bei der Röntgen-Brachytherapie handelt es sich um eine therapeutische Behandlung mit Röntgenstrahlen, bei der die Röntgenquelle sehr nahe an das zu behandelnde Gewebe, beispielsweise ein Tumor oder eine Gefäßwand nach der Durchführung einer endovaskulären Dilatation, gebracht wird. Um die Röntgenquelle entweder ohne oder mit möglichst geringem invasiven Eingriff im Inneren eines Körpers mit Hilfe eines Katheters oder einer Sonde einführen zu können, wird eine miniaturisierte Röntgenquelle benötigt, wie sie beispielsweise aus der US 6,721,392 B1 bekannt ist. Diese ist am distalen Ende einer Sonde angeordnet, die intraoperativ beispielsweise in einem Tumor oder nach dessen Entfernung in einem Tumorbett positioniert wird, wie es beispielsweise in der PR-Information der Carl-Zeiss AG, Medizintechnik Innovation von Carl Zeiss AG, "Intraoperative Strahlentherapie mit dem INTRABEAM System von der Carl Zeiss AG, Stand September 2004, näher erläutert ist.

Aus der US 2003/0149327 A1 ist eine miniaturisierte Röntgenquelle bekannt, die in einem Katheter angeordnet ist, mit dem sie in die Körperhohlräume (Lumen) eingeführt werden kann, um von dort aus ausgewählte Gewebezonen aus unmittelbarer Nähe zu bestrahlen. Sie enthält eine um die Achse des Katheters drehbare Abschirmung, um die Röntgenstrahlen gezielt im Wesentlichen senkrecht zur Achse in einen ausgewählten Raumwinkel abzustrahlen. Mit einer im Katheter angeordneten optischen Beobachtungseinrichtung kann die Umgebung des Katheters betrachtet werden. Hierzu wird eine Lichtquelle verwendet, die nur den Teil der Oberfläche des Hohlraumes beleuchtet, der auch bestrahlt wird.

Auch bei der endovaskulären Brachytherapie mit einem in der Spitze eines Katheters angeordneten Beta- oder Gammastrahler ist es beispielsweise aus der DE 10 2004 008 373 B3 bekannt, im Katheter eine optische Beobachtungseinrichtung anzuordnen. Hierzu wird ein Brachytherapie-Katheter mit einem auf der Grundlage der optischen Kohärenztomographie (OCT) arbeitenden OCT-Katheter zu einer Einheit integriert.

Wesentlich für den therapeutischen Erfolg ist, dass die von der Röntgenquelle in ein Bestrahlungsgebiet außerhalb des Katheters abgestrahlten Röntgenstrahlen weitgehend ausschließlich auf das zu behandelnde Gewebe, beispielsweise den Tumor, auftreffen, um eine möglichst geringe Belastung des daneben befindlichen gesunden Gewebes sicherzustellen. Dies erfordert eine präzise Positionierung des Bestrahlungsgebietes, d.h. eine präzise Positionierung und Ausrichtung der Röntgenquelle bzw. des Raumwinkels, in den die Röntgenstrahlen austreten.

Der Erfindung liegt nun die Aufgabe zu Grunde, eine Einrichtung für die Röntgen-Brachytherapie mit einer in das Innere eines Körpers einführbaren Sonde anzugeben, mit der eine genaue Positionierung des Bestrahlungsgebietes möglich ist.

Die genannte Aufgabe wird gelöst mit einer Einrichtung mit den Merkmalen des Patentanspruches 1. Gemäß diesen Merkmalen enthält die Einrichtung eine in das Innere eines Körpers einführbare Sonde, die an ihrem distalen Ende eine Röntgenquelle aufweist, die ein Röntgenstrahlbündel in ein Bestrahlungsgebiet außerhalb der Sonde abstrahlt, sowie eine optische Beobachtungseinrichtung zum Erzeugen eines zumindest einen Teil des Bestrahlungsgebietes wiedergebenden optischen Bildes, das im optischen Bild durch eine Markierung kenntlich gemacht ist. Dies ermöglicht eine genaue Positionierung des Bestrahlungsgebietes, d.h. eine genaue Positionierung der Röntgenquelle und des Raumwinkelbereiches, in dem die von der Röntgenquelle erzeugten Röntgenstrahlen abgestrahlt werden.

In der Sonde ist wenigstens eine der Röntgenquelle zugeordnete Lichtquelle angeordnet, die Licht aussendet, das das Strahlungsgebiet intrakorporal markiert. Hierzu sind eine Mehrzahl von Lichtquellen vorgesehen, die jeweils ein annähernd paralleles Strahlenbündel aussenden, das sich zumindest annähernd entlang der Randstrahlen des Röntgenstrahlbündels ausbreitet. Auf diese Weise kann der Wirkbereich der auf die Oberfläche der zu behandelnden Gewebezone auftreffenden Röntgenstrahlung besonders genau und unabhängig von der Lage und der Gestalt der Oberfläche der zu behandelnden Gewebezone wiedergegeben werden.

Unter dem Begriff "Sonde" ist im Folgenden allgemein ein Instrument zu verstehen, das in das Innere eines Körpers eingebracht werden kann. Dies kann sowohl ein Katheter im engeren Sinn sein, der in Hohlräume des Körpers (transluminal) eingeführt wird, als auch ein innerhalb einer Gewebezone (perkutan oder interstitiell) platzierbares nadelähnliches Instrument sein.

Wenn die optische Beobachtungseinrichtung eine nach dem OCT-Verfahren arbeitende Bildgebungseinrichtung umfasst, werden Gewebestrukturen im Sichtfeld der Beobachtungseinrichtung besonders deutlich wiedergegeben.

Wenn die optische Achse der Beobachtungseinrichtung im Objektraum mit der Mittenachse des Röntgenstrahlbündels zusammenfällt ist sichergestellt, dass die Bildmitte zugleich die Lage der Mittenachse des Röntgenstrahlbündels wiedergibt. Dann lässt sich der Wirkbereich der Röntgenstrahlen, beispielsweise bei einem kegelförmigen Röntgenstrahlbündel als Kreislinie in das Bild einblenden.

Wenn das Bestrahlungsgebiet relativ zur Sonde einstellbar ist, ist eine besonders hohe therapeutische Flexibilität gewährleistet. Wenn außerdem in einer Steuer- und Auswerteeinrichtung eine Software zur Bildauswertung sowie zum automatischen, gegebenenfalls sukzessiven Positionieren des Bestrahlungsgebietes derart vorgesehen ist, dass ein vorher im optischen Bild markiertes Behandlungsgebiet mit vorgebbaren Röntgenparametern bestrahlt wird, sind Fehlbedienungen während der therapeutischen Behandlung weitgehend vermieden.

In einer besonders bevorzugten Ausgestaltung der Einrichtung ist in der Steuer- und Auswerteeinrichtung eine Software zur automatischen Lokalisierung und Markierung des Behandlungsgebietes implementiert.

Eine Einrichtung gemäß der Erfindung ist insbesondere zum Einführen in eine Harnröhre oder einen Harnleiter und zur therapeutischen Behandlung eines Prostata-, Blasen- oder Nierentumors geeignet.

Wenn ein Marker oder ein Leuchtstoff appliziert wird, der sich bevorzugt in einem Tumor anlagert, und der durch Anregung mit elektromagnetischer Strahlung im optischen Bild sichtbar gemacht wird, kann das Bestrahlungsgebiet auf besonders einfache und anschauliche Weise korrekt positioniert werden, indem das Bestrahlungsgebiet mit dem durch den Leuchtstoff markierten Gebiet zur Deckung gebracht wird.

Wenn ein röntgensensitiver Farbstoff appliziert wird, der sich in einem Tumor lagert und sich bei einer Bestrahlung mit Röntgenstrahlen verfärbt, wird sichtbar, welche Areale des Tumors bereits behandelt wurden.

Die Einrichtung gemäß der Erfindung ist insbesondere bei der Behandlung eines Prostata-, Blasen- oder Nierentumors geeignet, bei der die Sonde in eine Harnröhre oder einen Harnleiter eingeführt wird.

Zur weiteren Erläuterung der Erfindung wird auf das Ausführungsbeispiel der Zeichnung verwiesen. Es zeigen:
- Fig. 1: eine Einrichtung gemäß der Erfindung in einer sche- matischen Prinzipdarstellung,
- Fig. 2-4: jeweils ein optisches Bild, in dem das Bestrah- lungsgebiet durch eine Markierung kenntlich gemacht ist.

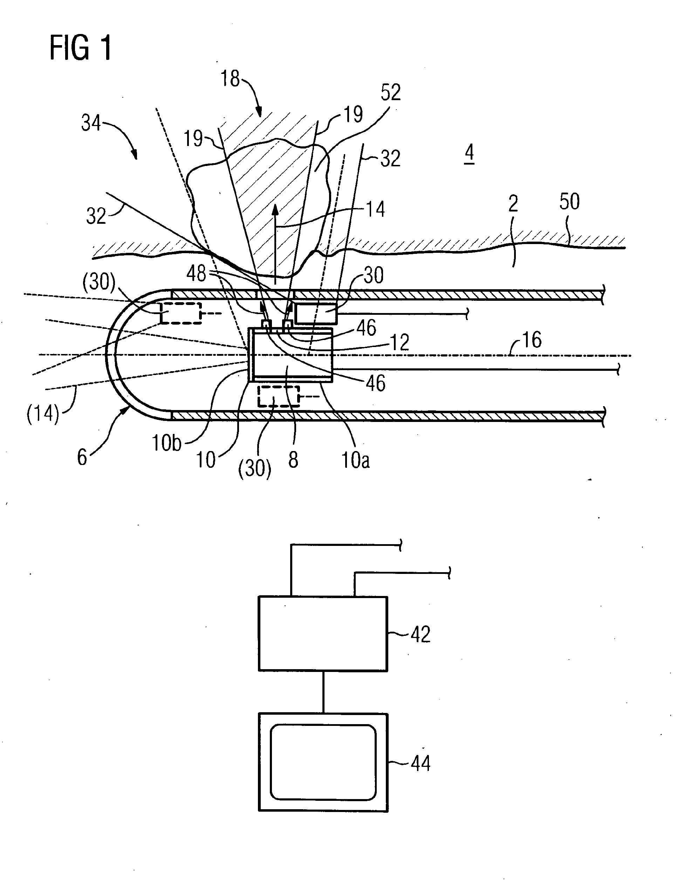

Gemäß Fig. 1 ist in einen Hohlraum (Lumen) 2 eines Körpers 4, bei dem es sich um eine Urethra (Harnröhre) oder einen Ureter (Harnleiter) handeln kann, eine Sonde 6, im Beispiel ein Katheter, eingeführt, in der an ihrem distalen Ende eine Röntgenquelle 8 angeordnet ist. Der Röntgenquelle 8 ist eine Abschirmung 10 zugeordnet, die im Ausführungsbeispiel einen zylindrischen Teil 10a enthält, der in seinem Umfang mit einer Blende oder Öffnung 12 versehen ist, durch die ein Röntgenstrahlbündel 14 senkrecht zur Längsachse 16 der Sonde 6, d.h. radial in ein durch die Form der Öffnung 12 und deren Abstand zur Anode der Röntgenquelle 8 definiertes, beispielsweise kegelförmiges Bestrahlungsgebiet 18 austreten kann, das in der Figur durch eine Schraffur hervorgehoben und durch Randstrahlen 19 kenntlich gemacht ist.

Der zylindrische Teil 10a der Abschirmung 10 ist innerhalb der Sonde 6 um deren Längsachse 16 drehbar angeordnet, so dass das Bestrahlungsgebiet 18 ebenfalls um diese Längsachse 16 geschwenkt werden kann.

Die Abschirmung 10 weist an einer ihrer Stirnseiten eine Stirnplatte 10b auf, die mit einer in der Figur nicht näher dargestellten verschließbaren Blende versehen ist, mit der es möglich ist, wahlweise ein Röntgenstrahlbündel 14 in Richtung der Längsachse 16 abzustrahlen. In diesem Fall ist entweder ein beweglicher Verschluss vorgesehen, mit dem die Öffnung 12 verschlossen werden kann oder eine Zusatzabschirmung, die in der Sonde 6 derart angeordnet ist, dass die Öffnung 12 im Bereich dieser Abschirmung positioniert werden kann.

In unmittelbarer Nähe der Röntgenquelle 8 im Bereich der Öffnung 12 ist eine insbesondere nach dem OCT-Verfahren arbeitende optische Beobachtungseinrichtung 30 mit einem durch Begrenzungslinien 32 veranschaulichten Sichtfeld 34 angeordnet, mit der ein Objektbereich beobachtet werden kann, der zumindest einen Teil des Bestrahlungsgebietes 18 wiedergibt.

Die Sonde 6 ist an eine Steuer- und Auswerteeinrichtung 42 angeschlossen, mit der die Röntgenquelle 8 und die Beobachtungseinrichtung 30 gesteuert und die von der Beobachtungseinrichtung 30 übermittelten Signale ausgewertet werden, so dass sie als optisches Bild in einer Wiedergabeeinrichtung 44, beispielsweise ein Monitor, dargestellt werden können.

Der Röntgenquelle 8 sind eine Mehrzahl von Lichtquellen 46 zugeordnet, die im Bereich der Öffnung 12 angeordnet sind und jeweils ein annähernd paralleles Strahlenbündel 48 aussenden, die sich jeweils entlang der Randstrahlen 19 des aus der Öffnung 12 austretenden Röntgenstrahlbündels 14 ausbreiten. Die von den Lichtquellen 46 emittierten Strahlenbündel, vorzugsweise Laserstrahlen, treffen auf die Oberfläche des Hohlraumes 2 auf, werden von der Beobachtungseinrichtung 30 erfasst und markieren auf diese Weise den Rand des Bestrahlungsgebietes 18.

In der Figur ist außerdem durch zwei gestrichelt eingezeichnete Beobachtungseinrichtungen 30 veranschaulicht, dass entweder eine Mehrzahl von Beobachtungseinrichtungen 30 vorgesehen ist oder aber eine Beobachtungseinrichtung 30 in unterschiedliche Positionen innerhalb der Sonde manövrierbar ist.

Die Beobachtungseinrichtung 30 ist entweder frei beweglich, d.h. unabhängig von der Abschirmung 10a in der Sonde 6 angeordnet. Alternativ hierzu kann sie auch mechanisch an die Abschirmung 10a gekoppelt sein, so dass sie beim Schwenken des Röntgenstrahlbündels mit der Abschirmung 10 mitbewegt wird.

Im Beispiel der Fig. 1 befindet sich im Bereich der Wand 50 des Hohlraumes 2 eine therapeutisch zu behandelnde Gewebezone 52, beispielsweise ein Tumor, insbesondere ein Prostatatumor, ein Blasentumor oder ein Nierentumor, die mit den Röntgenstrahlen 14 bestrahlt werden soll.

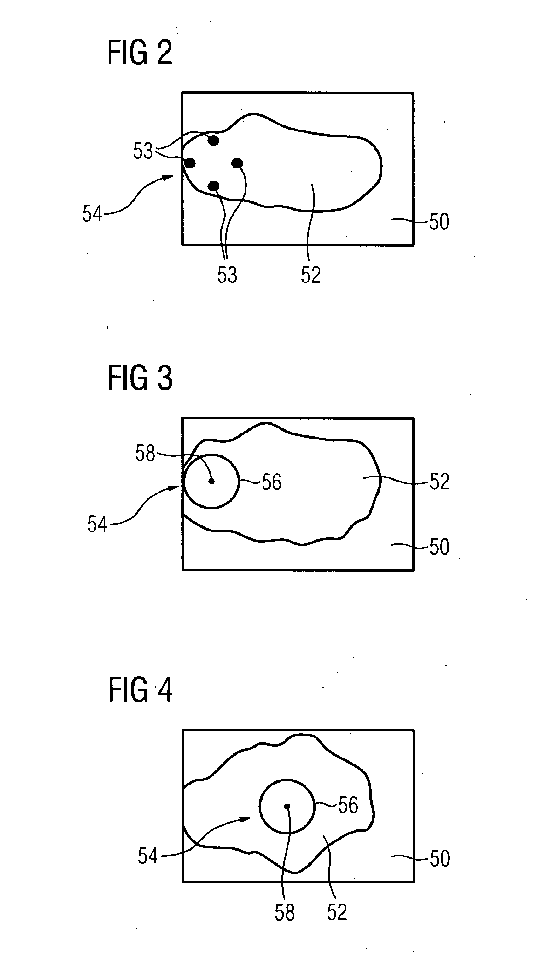

Gemäß Fig. 2 ist im optischen Bild aus der Umgebung der Sonde bei entsprechender Beleuchtung die Wand 50 des Hohlraumes mit der Gewebezone 52 sichtbar. Im Bild sind außerdem vier Lichtpunkte 53 erkennbar, wie sie beispielsweise von vier Lichtquellen 46 erzeugt werden, und die eine unmittelbare Markierung 54 der Schnittfläche des Bestrahlungsgebietes 18 mit der Wand 50 des Hohlraumes 2 darstellen.

Wird zusätzlich ein Leuchtstoff oder Marker appliziert, beispielsweise unmittelbar mit Hilfe der Sonde, der sich bevorzugt in der Gewebezone 52 anlagert, wenn es sich bei dieser um einen Tumor handelt, und der durch elektromagnetische Strahlung, beispielsweise durch die zur Beleuchtung verwendete Lichtquelle angeregt wird und Fluoreszenzlicht im sichtbaren Bereich emittiert, ist die Erkennbarkeit des Tumors zumindest in seinem Oberflächenbereich, mit dem er an den Hohlraum angrenzt, deutlich verbessert, so dass er auch automatisch mit einer Bilderkennungssoftware lokalisiert und eingegrenzt werden kann. Auf diese Weise ist bei ebenfalls bekannter Lage des Bestrahlungsgebietes, das im Beispiel anhand der Lichtpunkte 53 ebenfalls durch die Bilderkennungssoftware identifiziert werden kann, eine weitgehend automatisierte Positionierung der Sonde und therapeutische Behandlung des Tumors mit Hilfe von Röntgenparametern (Dosisleistung, Anodenstrom, Röhrenspannung und Bestrahlungsdauer) möglich, die vom Therapeuten anhand des ihm vorliegenden Befundes vorgegeben worden sind.

Alternativ hierzu kann das Behandlungsgebiet vom Therapeuten auch manuell markiert werden. Auch in diesem Fall kann die therapeutische Behandlung weitgehend automatisiert werden, indem das Bestrahlungsgebiet nach erfolgter manueller Markierung bei ruhender Sonde automatisch gegebenenfalls mehrfach positioniert wird, so dass das Behandlungsgebiet vollständig erfasst wird.

Alternativ zu der unmittelbaren Markierung gemäß Fig. 2 kann die Markierung 54 auch mit einer in der Steuer- und Auswerteeinrichtung implementierten bildverarbeitenden Software elektronisch in das Bild eingeblendet werden. Gemäß Fig. 3 kann dann die Einhüllende des auf die Oberfläche der Wand 50 auftreffenden Röntgenstrahlbündels als Kreislinie 56 und dessen Mittenachse als Punkt 58 eingeblendet werden.

In Fig. 4 ist ein Ausführungsbeispiel veranschaulicht, bei dem die Mittenachse des Sichtfeldes der Beobachtungseinrichtung 30 mit der Mittenachse des Röntgenstrahlbündels zusammenfällt. In diesem Fall ist eine genaue Positionierung vereinfacht, da Fehler, die durch schiefwinklige und versetzte Mittenachsen verursacht werden können, vermieden sind.

Die Erfindung ist anhand eines in den Hohlraum eines Körpers eingeführten Katheters dargestellt. Grundsätzlich ist die Erfindung auch für Sonden geeignet, die unmittelbar in das Gewebe eingeführt werden, wie das bei der eingangs erläuterten invasiven Nachbehandlung eines Tumorbettes eines vorher entfernten Tumors der Fall ist. Bei der zu behandelnden Gewebezone kann es sich auch um eine Gefäßwand handeln, die nach der Durchführung einer Dilatation zur Verringerung der Restenoserate bestrahlt werden soll.

## Patentansprüche

1. Einrichtung für die Röntgen-Brachytherapie mit einer in das Innere eines Körpers (4) einführbaren Sonde (6), die an ihrem distalen Ende eine Röntgenquelle (8) aufweist, die ein Röntgenstrahlbündel (14) in ein Bestrahlungsgebiet (18) außerhalb der Sonde (6) abstrahlt, und mit einer in der Sonde (6) angeordneten optischen Beobachtungseinrichtung (40) zum Erzeugen eines zumindest einen Teil des Bestrahlungsgebietes (18) wiedergebenden optischen Bildes, **dadurch gekennzeichnet, dass** das Bestrahlungsgebiet (18) im optischen Bild durch eine Markierung (54) **dadurch** kenntlich gemacht ist, dass in der Sonde (6) mehrere der Röntgenquelle (8) zugeordnete Lichtquellen (46) angeordnet sind, die jeweils ein annähernd paralleles Strahlenbündel (48) aussenden, das sich zumindest annähernd entlang der Randstrahlen (19) des Röntgenstrahlbündels (18) ausbreitet und auf diese Weise das Bestrahlungsgebiet (18) intrakorporal markieren.

2. Einrichtung nach Anspruch 1, bei der die optische Beobachtungseinrichtung (40) eine nach dem OCT-Verfahren arbeitende Bildgebungseinrichtung umfasst.

3. Einrichtung nach Anspruch einem der vorhergehenden Ansprüche, bei der die optische Achse der Beobachtungseinrichtung (30) im Objektraum mit der Mittenachse des Röntgenstrahlbündels (14) zusammenfällt.

4. Einrichtung nach einem der vorhergehenden Ansprüche, bei der das Bestrahlungsgebiet (18) relativ zur Sonde (6) einstellbar ist.

5. Einrichtung nach einem der vorhergehenden Ansprüche, mit einer in einer Steuer- und Auswerteeinrichtung (42) implementierten Software zur Bildauswertung sowie zum automatischen, gegebenenfalls sukzessiven Positionieren des Bestrahlungsgebietes (18) derart, dass ein vorher im optischen Bild markiertes Behandlungsgebiet mit vorgebbaren Röntgenparametern bestrahlt wird.

6. Einrichtung nach Anspruch 5, mit einer Software zur automatischen Lokalisierung und Markierung des Behandlungsgebietes.

## Claims

1. Device for X-ray brachytherapy with a probe (6) that can be introduced into the interior of a body (4), which probe has an X-ray source (8) at the distal end thereof, the X-ray source emitting an X-ray beam bundle (14) into an exposure area (18) outside of the probe (6), and with an optical observation device (30), arranged in the probe (6), for generating an optical image reproducing at least part of the exposure area (18), **characterized in that** the exposure area (18) is identified by a marking (54) in the optical image by virtue of the fact that a plurality of light sources (46) associated with the X-ray source (8) are arranged in the probe (6), which light sources respectively emit an approximately parallel bundle of rays (48) that propagates at least approximately along the boundary rays (19) of the X-ray beam bundle (14) and thereby, in the body, marks the exposure area (18).

2. Device according to Claim 1, in which the optical observation device (30) comprises an imaging device operating according to the OCT method.

3. Device according to one of the preceding claims, in which the optical axis of the observation device (30) coincides with the centre axis of the X-ray beam bundle (14) in the object space.

4. Device according to one of the preceding claims, in which the exposure area (18) can be adjusted relative to the probe (6).

5. Device according to one of the preceding claims, with software, implemented in a control and evaluation device (42), for image evaluation and for automated, possibly successive, positioning of the exposure area (18) such that a treatment area marked previously in the optical image is irradiated with predeterminable X-ray parameters.

6. Device according to Claim 5, with software for automated localizing and marking of the treatment area.

## Revendications

1. Dispositif de brachythérapie aux rayons X comprenant une sonde ( 6 ) pouvant être introduite à l'intérieur d'un corps ( 4 ) et ayant à son extrémité distale une source ( 8 ) de rayons X, qui est émet un faisceau ( 14 ) de rayons X dans une région ( 18 ) d'irradiation à l'extérieur de la sonde ( 6 ) et comprenant un dispositif ( 40 ) optique d'observation disposé dans la sonde ( 6 ), pour la production d'une image optique reproduisant au moins une partie de la région ( 18 ) d'irradiation, **caractérisé en ce que** la région ( 18 ) d'irradiation est rendue reconnaissable dans l'image optique par un repère ( 54 ) par le fait qu'il est disposé dans la sonde ( 6 ) plusieurs sources ( 46 ) lumineuses associées à la source ( 8 ) de rayons X, qui émettent respectivement un faisceau ( 48 ) de rayonnement à peu près parallèle, qui se propage au moins à peu près le long des rayons ( 19 ) de bord du faisceau ( 18 ) de rayons X et repère ainsi intra-corporellement la région ( 18 ) d'irradiation.

2. Dispositif suivant la revendication 1, dans lequel le dispositif ( 40 ) optique d'observation comprend un dispositif de formation d'images opérant suivant le procédé OCT.

3. Dispositif suivant l'une des revendications précédentes, dans lequel l'axe optique du dispositif ( 30 ) d'observation coïncide dans l'espace objet avec l'axe médian du faisceau ( 14 ) de rayons X.

4. Dispositif suivant l'une des revendications précédentes, dans lequel la région ( 18 ) d'irradiation est réglable par rapport à la sonde ( 6 ).

5. Dispositif suivant l'une des revendications précédentes, comprenant un logiciel mis en oeuvre dans un dispositif ( 42 ) de commande et d'exploitation pour exploiter l'image, ainsi que pour mettre en position automatiquement, le cas échéant successivement, la région ( 18 ) d'irradiation, de manière à ce qu'une région de traitement repérée auparavant dans l'image optique soit irradiée avec des paramètres de rayons X pouvant être prescrits.

6. Dispositif suivant la revendication 5, comprenant un logiciel de localisation automatique et de repérage de la région de traitement.
